# EUROPEAN PATENT SPECIFICATION

(11) **EP 4 185 575 B1**
(45) Date of publication and mention of the grant of the patent: **28.08.2024**
(21) Application number: 21749128.1
(22) Date of filing: 21.07.2021
(51) Int. Cl.: C07D 401/14, C07D 413/14, A61K 31/4439, A61P 25/00

(54) **HETEROARYL-METHYL SUBSTITUTED TRIAZOLES AS VASOPRESSIN RECEPTOR V1A ANTAGONISTS**
HETEROARYLMETHYLSUBSTITUIERTE TRIAZOLE ALS VASOPRESSINREZEPTOR-V1A-ANTAGONISTEN
TRIAZOLES SUBSTITUÉS PAR HÉTÉROARYLE-MÉTHYLE EN TANT QU'ANTAGONISTES DU RÉCEPTEUR V1A DE LA VASOPRESSINE

(30) Priority: 23.07.2020 EP 20187298
(43) Date of publication of application: 31.05.2023
(73) Proprietor: F. Hoffmann-La Roche AG, 4070 Basel (CH)
(72) Inventor: GALLEY, Guido, 4070 Basel (CH); SCHNIDER, Patrick, 4070 Basel (CH)
(74) Representative: Jochnowitz, Evan
(86) International application number: PCT/EP2021/070310
(87) International publication number: WO 2022/018105

(56) References cited:
- WO-A1-2005/079808
- WO-A1-2005/105779
- WO-A1-2006/114706
- WO-A1-2006/123242
- WO-A1-2011/131596
- WO-A1-2013/050334
- WO-A1-2015/024819

## Description

The present invention provides heteroaryl-methyl substituted triazoles, which act as V1a receptor modulators, and in particular as V1a receptor antagonists, their manufacture, pharmaceutical compositions containing them and their use as medicaments. The present compounds are useful as therapeutics acting peripherally and centrally in the conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

### Technical Field

The present invention provides a compound of formula I, 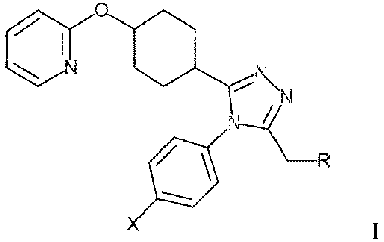 wherein the substituents and variables are as described below and in the claims, or a pharmaceutically acceptable salt thereof.

A method is provided for antagonizing the V1a receptor, the method comprising contacting the receptor with an effective amount of a compound of formula I as described herein, or a pharmaceutically acceptable salt thereof, or a pharmaceutical composition comprising the same.

A method is provided for treatment of a malcondition in a subject for which antagonism of the V1a receptor is medically indicated. Such method comprises administering to the subject an effective amount of a compound of formula I as described herein, or a pharmaceutically acceptable salt thereof, or a pharmaceutical composition comprising the same, at a frequency and for duration sufficient to provide a beneficial effect to the subject.

In an embodiment, a pharmaceutical composition is provided comprising a compound of formula I as described herein, or a pharmaceutically acceptable salt thereof, in combination with at least one pharmaceutically acceptable carrier, diluent, or excipient.
In an embodiment, a method of synthesis is provided for a compound of formula I as described herein, or a pharmaceutically acceptable salt thereof.

The present compounds are V1a receptor antagonists, useful for the treatment of autistic spectrum disorders, social anxiety disorder, post-traumatic stress disorder (PTSD), brain edema in stroke or traumatic brain injury, and phase shift sleep disorders, in particular jetlag.

### Background Art

Three vasopressin receptors, all belonging to the class I G-protein coupled receptors, are known. The V1a receptor is expressed in the brain, liver, vascular smooth muscle, lung, uterus and testis, the V1b or V3 receptor is expressed in the brain and pituitary gland, the V2 receptor is expressed in the kidney where it regulates water reabsorption and mediates the antidiuretic effects of vasopressin (Robben, *et al*.)ⁱ*.* Compounds with activity at the V2 receptor may therefore cause side-effects on blood homeostasis.

The oxytocin receptor is related to the Vasopressin receptor family and mediates the effects of the neurohormone oxytocin in the brain and the periphery. Oxytocin is believed to have central anxiolytic effects (Neumann)ⁱⁱ. Central oxytocin receptor antagonism might therefore lead to anxiogenic effects, which are regarded as undesired side-effects.

In the brain vasopressin acts as a neuromodulator and is elevated in the amygdala during stress (Ebner, *et al*.)ⁱⁱⁱ. It is known that stressful life events can trigger major depression and anxiety (Kendler, *et al*.)^{iv} and that both have very high comorbidity, with anxiety often preceding major depression (Regier, *et al*.)^{v}*.* The V1a receptor is extensively expressed in the brain and particularly in limbic areas like the amygdala, lateral septum and hippocampus which are playing an important role in the regulation of anxiety. Indeed V1a knock-out mice show a reduction in anxious behavior in the plus-maze, open field and light-dark box (Bielsky, *et al*.)^{vi}*.* The down-regulation of the V1a receptor using antisense oligonucleotide injection in the septum also causes a reduction in anxious behavior (Landgraf, *et al*.)^{vii}*.* Vasopressin or the V1a receptor are also implicated in other neuropsychological disorders: genetic studies linked sequence polymorphism in the promoter of the human V1a receptor to autistic spectrum disorders (Yirmiya, *et al*.)^{viii}*,* intranasal administration of vasopressin was shown to influence aggression in human males (Thompson, *et al*.)^{ix} and vasopressin levels were found to be elevated in schizophrenic patients (Raskind, *et al*.)^{x} and patients with obsessive-compulsive disorder (Altemus, *et al*.)^{xi}*.*

Autistic Spectrum Disorders (ASD) are a clinically heterogeneous condition characterized by defects in socialization and language. ASD include a wide range of abnormalities including a genuine incapacity to organize affective relations, behavioral anomalies in reciprocal social interactions, verbal and non-verbal communication, limited interest in the surrounding environment associated with stereotyped movements and repetitive plays (Bourreau *et al*, 2009)^{xii}. Research to date indicates that a genetic predisposition may be involved, but also environmental factors have to be taken into consideration (Bourgeron, 2009)^{xiii}. There is at present no efficient biological! pharmaceutical treatment to ASD.

The suprachiasmatic nucleus (SCN) is the endogenous clock of the body regulating circadian rhythmicity and is known to be rich in vasopressin neurons (Kalsbeek *et al.* 2010)^{xiv}, producing and releasing vasopressin with a 24h circadian rhythm (Schwartz *et al.* 1983)^{xv}. A major regulatory effect of vasopressin on circadian rhythm could not be demonstrated by the prior art. The Brattleboro rat, a rat strain naturally lacking vasopressin due to a point mutation, has no obvious defect in its circadian rhythm (Groblewski *et al.* 1981)^{xvi}. Injection of vasopressin directly in the hamster SCN had no effect on circadian phase shift (Albers *et al.* 1984)^{xvii}. In contrast, the vasopressin receptors were shown to modulate the circadian clock in a more subtle way. Yamaguchi *et al* (2013)^{xviii} demonstrated that V1a knock-out and V1a/V1b double knock-out mice show faster reentrainment to the new light/dark cycle after a circadian phase advance or a phase delay, an experiment mimicking jet-lag in humans. The same result was obtained after chronic administration of a mixture of V1a and V1b small molecule antagonists through a minipump directly on the SCN.

### Detailed description of the invention

The description may encompass subject-matter extending beyond the scope of the claims. However the scope of the invention and of protection is solely defined by the appended claims and shall not extend beyond their scope. In particular, the scope of protection shall not include any methods of therapeutic treatment of the human or animal body, even if disclosed or implied herein.

Object of the present invention is a compound of formula I as described herein and their pharmaceutically acceptable salts thereof, the preparation of the above mentioned compounds, medicaments containing them and their manufacture as well as the above mentioned compounds for use in a therapeutic and/or prophylactic treatment of diseases and disorders which are associated with modulation of the V1a receptor, and in particular with V1a receptor antagonism. A further object of the invention is to provide selective inhibitors of the V1a receptor, since selectivity for the V1a receptor is expected to afford a low potential to cause unwanted off-target related side effects such as discussed above.

The following definitions of the general terms used in the present description apply irrespectively of whether the terms in question appear alone or in combination with other groups.

The term "alkyl", alone or in combination with other groups, stands for a hydrocarbon radical which may be linear or branched, with single or multiple branching, wherein the alkyl group in general comprises 1 to 6 carbon atoms, for example, methyl (Me), ethyl (Et), propyl, isopropyl (i-propyl), n-butyl, i-butyl (isobutyl), 2-butyl (sec-butyl), t-butyl (tert-butyl), isopentyl, 2-ethyl-propyl, 1,2-dimethyl-propyl and the like. Particular "alkyl" groups have 1 to 4 carbon atoms ("alkyl"). A specific group is methyl.

The term "halogen", alone or in combination with other groups, denotes chloro (Cl), iodo (I), fluoro (F) and bromo (Br).

The term "halogen-alkyl" of "haloalkyl", alone or in combination with other groups, refers to alkyl as defined herein, which is substituted by one or multiple halogen, in particular 1-5 halogen, more particular 1-3 halogen. Particular halogen is chloro.

The term "hydroxy", alone or in combination with other groups, refers to -OH.

The term "hydroxy-alkyl", alone or in combination with other groups, denotes an alkyl group as defined above, with 1 to 6 carbon atoms, wherein at least one of the hydrogen atoms of the alkyl group is replaced by a hydroxy, i.e. by an OH group. An example for hydroxyalkyl is hydroxyethyl.

The term "cyano" denotes the group -CN.

The term "heterocycloalkyl", alone or in combination, denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Bicyclic means consisting of two cycles having two ring atoms in common, i.e. the bridge separating the two rings is either a single bond or a chain of one or two ring atoms.

The term "aryl", alone or in combination with other groups, denotes a monovalent cyclic aromatic hydrocarbon moiety consisting of a mono- or bicyclic aromatic ring. Preferred aryl are phenyl or naphthyl. Aryl may be unsubstituted or substituted as described herein.

The terms "heteroaryl", alone or in combination with other groups, refers to a monovalent aromatic containing from one to four ring heteroatoms selected from N and O, the remaining ring atoms being C. 5- or 6-membered heteroaryl are preferred. Examples for heteroaryl moieties include but are not limited to triazolyl, imidazolyl, oxyzolyl, pyrazolyl, and tetrazolyl. Heteroaryl may be unsubstituted or substituted as described herein.

When indicating the number of substituents, the term "one or more" means from one substituent to the highest possible number of substitution, i.e. replacement of one hydrogen up to replacement of all hydrogens by substituents. Thereby, one, two or three substituents are preferred. Even more preferred are one or two substituents or one substituent.

The term "pharmaceutically acceptable salts" refers to salts that are suitable for use in contact with the tissues of humans and animals. Examples of suitable salts with inorganic and organic acids are, but are not limited to acetic acid, citric acid, formic acid, fumaric acid, hydrochloric acid, lactic acid, maleic acid, malic acid, methane-sulfonic acid, nitric acid, phosphoric acid, p-toluenesulphonic acid, succinic acid, sulfuric acid, sulphuric acid, tartaric acid, trifluoroacetic acid and the like. Particular acids are formic acid, trifluoroacetic acid and hydrochloric acid. Particular are hydrochloric acid, trifluoroacetic acid and fumaric acid.

The terms "pharmaceutically acceptable carrier" and "pharmaceutically acceptable auxiliary substance" refer to carriers and auxiliary substances such as diluents or excipients that are compatible with the other ingredients of the formulation.

The term "pharmaceutical composition" encompasses a product comprising specified ingredients in pre-determined amounts or proportions, as well as any product that results, directly or indirectly, from combining specified ingredients in specified amounts. In particular, it encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients.

The term "half maximal inhibitory concentration" (IC₅₀) denotes the concentration of a particular compound required for obtaining 50% inhibition of a biological process in vitro. IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed. The IC₅₀ value can be converted to an absolute inhibition constant (Ki) using the Cheng-Prusoff equation (Biochem. Pharmacol. (1973) 22:3099). The term "inhibition constant" (Ki) denotes the absolute binding affinity of a particular inhibitor to a receptor. It is measured using competition binding assays and is equal to the concentration where the particular inhibitor would occupy 50% of the receptors if no competing ligand (e.g. a radioligand) was present. Ki values can be converted logarithmically to pKi values (-log Kᵢ), in which higher values indicate exponentially greater potency.

The term "hydrate" refers to a compound that exists in combination with water molecules. The combination can include water in stoichiometric quantities, such as a monohydrate or a dehydrate, or can include water in random amounts. As the term is used herein a "hydrate" refers to a solid form; that is, a compound in a water solution, while it may be hydrated, is not a hydrate as the term is used herein.

The term "solvate" refers to a hydrate except that a solvent other that water is present. For example, methanol or ethanol can form an "alcoholate", which can again be stoichiometric or non-stoichiometric. As the term is used herein a "solvate" refers to a solid form; that is, a compound in a solvent solution, while it may be solvated, is not a solvate as the term is used herein.

The term "antagonist" denotes a compound that diminishes or prevents the action of another compound as defined e.g. in Goodman and Gilman's "The Pharmacological Basis of Therapeutics, 7th ed." in page 35, Macmillan Publ. Company, Canada, 1985. In particular, antagonists refer to a compound that attenuates the effect of an agonist. A "competitive antagonist" binds to the same site of a receptor as the agonist but does not activate the receptor, thus blocks the agonist's action. A "non-competitive antagonist" binds to an allosteric (non-agonist) site on the receptor to prevent activation of the receptor. A "reversible antagonist" binds non-covalently to the receptor, therefore can be "washed out". An "irreversible antagonist" binds covalently to the receptor and cannot be displaced by either competing ligands or washing.

"Isotope" refers to atoms with the same number of protons but a different number of neutrons, and an isotope of a compound of formula I as described herein includes any such compound wherein one or more atoms are replaced by an isotope of that atom. For example, carbon 12, the most common form of carbon, has six protons and six neutrons, whereas carbon 13 has six protons and seven neutrons, and carbon 14 has six protons and eight neutrons. Hydrogen has two stable isotopes, deuterium (one proton and one neutron) and tritium (one proton and two neutrons). While fluorine has a number of isotopes, fluorine 19 is longest-lived. Thus, an isotope of a compound of formula I as described herein includes, but not limited to, compounds of formula I as described herein wherein one or more carbon 12 atoms are replaced by carbon-13 and/or carbon-14 atoms, wherein one or more hydrogen atoms are replaced with deuterium and/or tritium, and/or wherein one or more fluorine atoms are replaced by fluorine-19.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The term "as defined herein" and "as described herein" when referring to a variable incorporates by reference the broad definition of the variable as well as preferred, more preferred and most preferred definitions, if any.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

The term "aromatic" denotes the conventional idea of aromaticity as defined in the literature, in particular in IUPAC^{xix}.

The term "pharmaceutically acceptable excipient" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

The terms "Autistic Spectrum" and "Autistic Spectrum Disorders" summarize conditions classified as pervasive developmental disorders, which include but are not limited to autism, Asperger syndrome, pervasive developmental disorder not otherwise specified (PDD-NOS), childhood disintegrative disorder, Rett syndrome and Fragile X, in particular autism. These disorders are typically characterized by social deficits, communication difficulties, stereotyped or repetitive behaviors and interests, and cognitive delays.

The term "phase shift sleep disorders" summarizes conditions classified as disturbances in the circadian rhythm, i.e. the approximately 24-hour cycles that are generated by an organism, e.g. a human being. Phase shift sleep disorders include, but are not limited to transient disorders like jetlag or a changed sleep schedule due to work, social responsibilities, or illness, as well as chronic disorders like delayed sleep-phase syndrome (DSPS), delayed sleep-phase type (DSPT), advanced sleep-phase syndrome (ASPS), and irregular sleep-wake cycle.

In detail, aspect 1, the present invention provides compounds of the general formula I

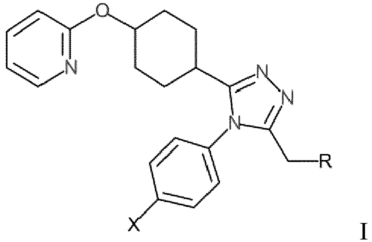

Wherein:
R is a heteroaryl ring, unsubstituted or substituted with H, halogen, alkyl, haloalkyl, cyano and hydroxyl;
X is H, halogen, alkyl or haloalkyl;
or a pharmaceutically acceptable salt thereof.

A certain embodiment of this invention, aspect 2, refers to a compound of formula I according to aspect 1, wherein R is a heteroaryl ring comprising 1-to-4 N and 0-to-1 O, unsubstituted or substituted with H, halogen, alkyl, haloalkyl, cyano and hydroxyl; or a pharmaceutically acceptable salt thereof.

A certain embodiment of this invention, aspect 3, refers to a compound of formula I according to aspects 1 to 2, wherein R is a 5-membered heteroaryl ring comprising 1-to-4 N and 0-to-1 O, unsubstituted or substituted with H, halogen, alkyl, haloalkyl, cyano and hydroxyl; or a pharmaceutically acceptable salt thereof.

A certain embodiment of this invention, aspect 4, refers to a compound of formula I according to aspects 1 to 3, wherein R is a 5-membered heteroaryl ring comprising 1-to-4 N and 0-to-1 O, unsubstituted or substituted with alkyl.

A certain embodiment of this invention, aspect 5, refers to a compound of formula I according to aspects 1 to 4, wherein R is a 5-membered heteroaryl ring comprising 1 N and 1 O either unsubstituted or substituted with methyl, or unsubstituted heteroaryl ring comprising 1-to-4 N.

A certain embodiment of this invention, aspect 6, refers to a compound of formula I according to aspects 1 to 5, wherein R is methylisoxazolyl, isoxazolyl, pyrazolyl, triazolyl, imidazolyl, or tetrazolyl.

A certain embodiment of this invention, aspect 7, refers to a compound of formula I according to aspects 1 to 6, wherein X is halogen.

A certain embodiment of this invention, aspect 8, refers to a compound of formula I according to aspects 1 to 7, wherein X is Cl.

In a certain embodiment of the invention, the compound of formula I is provided wherein:
R is a 5-membered heteroaryl ring comprising 1 N and 1 O either unsubstituted or substituted with methyl, or unsubstituted heteroaryl ring comprising 1-to-4 N;
X is halogen;
or a pharmaceutically acceptable salt thereof.

In a certain embodiment of this invention, the compound of formula I is provided wherein:
R is a 5-membered heteroaryl ring comprising 1 N and 1 O either unsubstituted or substituted with methyl, or unsubstituted heteroaryl ring comprising 1-to-4 N;
X is Cl;
or a pharmaceutically acceptable salt thereof.

In a certain embodiment of this invention, the compound of formula I is provided wherein:
R is methylisoxazolyl, isoxazolyl, pyrazolyl, triazolyl, imidazolyl, or tetrazolyl;
Xis Cl;
or a pharmaceutically acceptable salt thereof.

Examples for the compound according to the invention are shown in the experimental part and the table below.

| **Ex** | **Structure** | **Ex** | **Structure** |
|---|---|---|---|
| 1 | 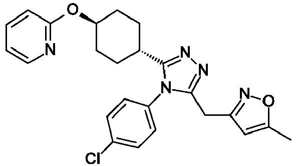 | 2 | 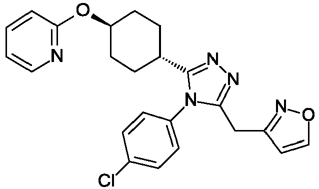 |
| 3 | 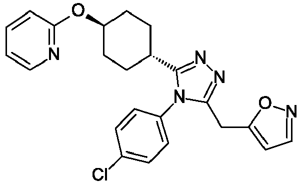 | 4 | 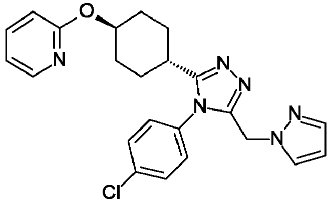 |
| 5 | 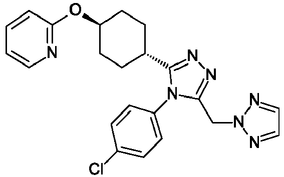 | 6 | 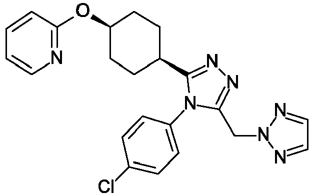 |
| 7 | 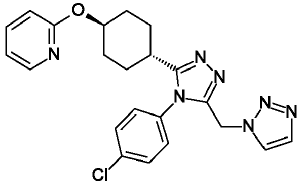 | 8 | 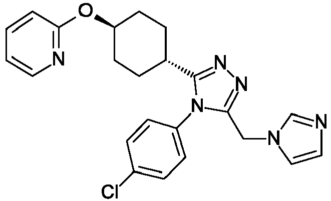 |
| 9 | 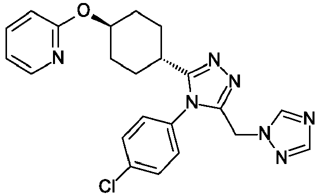 | 10 | 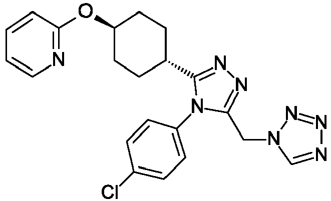 |

Preferred compounds of the invention are shown in the examples and listed below as:
trans-3-[[4-(4-chlorophenyl)-5-(4-pyridin-2-yloxycyclohexyl)-1,2,4-triazol-3-yl]methyl]-5-methyl-1,2-oxazole;
trans-3-[[4-(4-chlorophenyl)-5-(4-pyridin-2-yloxycyclohexyl)-1,2,4-triazol-3-yl]methyl]-1,2-oxazole;
trans-5-[[4-(4-chlorophenyl)-5-(4-pyridin-2-yloxycyclohexyl)-1,2,4-triazol-3-yl]methyl]-1,2-oxazole;
trans-2-[4-[4-(4-chlorophenyl)-5-(pyrazol-1-ylmethyl)-1,2,4-triazol-3-yl]cyclohexyl]oxypyridine;
trans-2-[4-[4-(4-chlorophenyl)-5-(triazol-2-ylmethyl)-1,2,4-triazol-3-yl]cyclohexyl]oxypyridine;
cis-2-[4-[4-(4-chlorophenyl)-5-(triazol-2-ylmethyl)-1,2,4-triazol-3-yl]cyclohexyl]oxypyridine;
trans-2-[4-[4-(4-chlorophenyl)-5-(triazol-1-ylmethyl)-1,2,4-triazol-3-yl]cyclohexyl]oxypyridine;
trans-2-[4-[4-(4-chlorophenyl)-5-(imidazol-1-ylmethyl)-1,2,4-triazol-3-yl]cyclohexyl]oxypyridine;
trans-2-[4-[4-(4-chlorophenyl)-5-(1,2,4-triazol-1-ylmethyl)-1,2,4-triazol-3-yl]cyclohexyl]oxypyridine;
trans-2-[4-[4-(4-chlorophenyl)-5-(tetrazol-1-ylmethyl)-1,2,4-triazol-3-yl]cyclohexyl]oxypyridine;
or a pharmaceutically acceptable salt thereof.

Particularly preferred are:
trans-3-[[4-(4-chlorophenyl)-5-(4-pyridin-2-yloxycyclohexyl)-1,2,4-triazol-3-yl]methyl]-5-methyl-1,2-oxazole;
trans-3-[[4-(4-chlorophenyl)-5-(4-pyridin-2-yloxycyclohexyl)-1,2,4-triazol-3-yl]methyl]-1,2-oxazole;
trans-5-[[4-(4-chlorophenyl)-5-(4-pyridin-2-yloxycyclohexyl)-1,2,4-triazol-3-yl]methyl]-1,2-oxazole;
trans-2-[4-[4-(4-chlorophenyl)-5-(pyrazol-1-ylmethyl)-1,2,4-triazol-3-yl]cyclohexyl oxypyridine;
trans-2-[4-[4-(4-chlorophenyl)-5-(triazol-2-ylmethyl)-1,2,4-triazol-3-yl]cyclohexyl oxypyridine;
cis-2-[4-[4-(4-chlorophenyl)-5-(triazol-2-ylmethyl)-1,2,4-triazol-3-yl]cyclohexyl oxypyridine;
or a pharmaceutically acceptable salt thereof.

In a certain embodiment, the invention relates to a process to manufacture a compound of formula I comprising the step of reacting a compound of formula II with a compound of formula III, wherein X and R are as defined above.

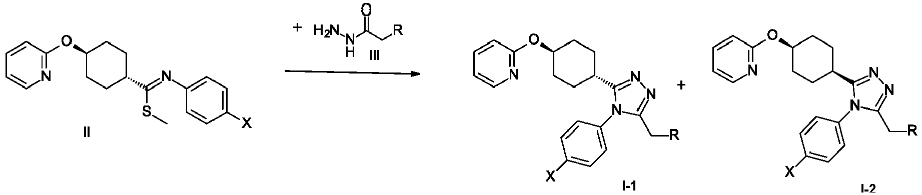

A certain embodiment of this invention refers to a compound of formula I as described herein, when manufactured by a process as defined herein.

A certain embodiment of this invention refers to a compound of formula I as described herein for use as therapeutically active substance.

A certain embodiment of this invention refers to a pharmaceutical composition comprising a compound of formula I as described herein and a pharmaceutically acceptable carrier and/or a pharmaceutically acceptable auxiliary substance or excipient.

A certain embodiment of this invention refers to a compound of formula I as described herein for the use as therapeutically active substance for the therapeutic and/or prophylactic treatment of diseases and disorders which are associated with V1a receptor antagonism.

A method is provided for treatment of a vasopressin-dependent condition, whether organic, stress-induced or iatrogenic.

The terms "vasopressin-dependent condition" refer to conditions related to inappropriate secretion of vasopressin, particularly in the response to chronic stress and in circuits that are dysregulated in affective disorders, such as disorders of stress, mood, and behavioral disorders, including stress-related affective disorders. Vasopressin-dependent conditions, include conditions such as cardiovascular conditions, for example hypertension, pulmonary hypertension, cardiac insufficiency, myocardial infarction or coronary vasospasm, in particular in smokers, Raynaud's syndrome, unstable angina and PTCA (percutaneous transluminal coronary angioplasty), cardiac ischemia, hemostasis disturbances or thrombosis; conditions of the central nervous system, such as migraine, cerebral vasospasm, cerebral hemorrhage, trauma and cerebral edema, depression, anxiety, stress, emotional disorders, obsessive-compulsive disorder, panic attacks, psychotic states, aggression, memory or sleep disorders, or cognitive disorders, for example disorders associated with impaired social cognition (e.g., schizophrenia, autism spectrum disorder); conditions of the renal system, such as renal vasospasm, necrosis of the renal cortex, nephrogenic diabetes insipidus or diabetic nephropathy; or conditions of the gastric system, such as gastric vasospasm, cirrhosis of the liver, ulcers or the pathology of vomiting, for example nausea, including nausea due to chemotherapy, or travel sickness; circadian rhythm-related disorders such as phase shift sleep disorders, jet-lag, sleep disorders and other chronobiological disorders. Additional examples of vasopressin-dependent conditions include but are not limited to neuropsychiatric disorders, neuropsychiatric symptoms in neurodegenerative diseases, PTSD, inappropriate aggression, anxiety, depressive disorders, major depression, obsessive compulsive disorder, autistic spectrum disorders, schizophrenia, and aggressive behavior, and other affective disorders.

A a method is provided for treatment of an autism spectrum disorder, comprising administering to a subject in need thereof an effective amount of a compound of formula I, or a pharmaceutically acceptable salt thereof, or a pharmaceutical composition comprising the same, at a frequency and for a duration sufficient to provide a beneficial effect to the subject.

Autism spectrum disorder (ASD), also referred to herein as autistic spectrum disorders, is a blanket term describing a complex developmental disorder that affects the brain's normal development of social and communication skills. Core symptoms of ASD include impaired social interactions such as social interaction difficulties, communication challenges including impaired verbal and nonverbal communication, problems processing information from the senses, and a tendency to engage in restricted and repetitive patterns of behavior. In one embodiment, the core symptoms of the autism spectrum disorder are impaired social interactions and communication challenges. In one embodiment, the core symptom of the autism spectrum disorder is impaired social interactions. In one embodiment, the core symptom of the autism spectrum disorder is impaired communication challenges. In one embodiment, the core symptom of the autism spectrum disorder is the tendency to engage in restricted and repetitive patterns of behavior.

A method is provided for treatment of an anxiety disorder, comprising administering to a subject in need thereof an effective amount of a compound of formula I as described herein, or a pharmaceutically acceptable salt thereof, or a pharmaceutical composition comprising the same, at a frequency and for a duration sufficient to provide a beneficial effect to the subject.

Anxiety disorder is a blanket term covering several different forms of abnormal and pathological fear and anxiety. Current psychiatric diagnostic criteria recognize a wide variety of anxiety disorders, including generalized anxiety disorder, panic disorder, stress-related disorders, obsessive compulsive disorder, phobia, social anxiety disorder, separation anxiety disorder and post-traumatic stress disorder (PTSD). In one embodiment, the anxiety disorder is a social anxiety disorder. In one embodiment, the anxiety disorder is phobia. In one embodiment, the anxiety disorder is a stress-related disorder. In one embodiment, the anxiety related disorder is PTSD.

Generalized anxiety disorder is a common chronic disorder characterized by long-lasting anxiety that is not focused on any one object or situation. A person suffering from generalized anxiety experience non-specific persistent fear and worry and become overly concerned with everyday matters. Generalized anxiety disorder is the most common anxiety disorder to affect older adults.

In panic disorder, a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attack's potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

Obsessive compulsive disorder is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of Phobia, which includes all cases in which fear and anxiety is triggered by a specific stimulus or situation. Sufferers typically anticipate terrifying consequences from encountering the object of their fear, which can be anything from social phobia, specific phobia, agoraphobia, phobia of an animal to a location to a bodily fluid.

Post-traumatic stress disorder or PTSD is an anxiety disorder which results from a traumatic experience. Post-traumatic stress can result from an extreme situation, such as combat, rape, hostage situations, or even serious accident. It can also result from long term (chronic) exposure to a severe stressor, for example soldiers who endure individual battles but cannot cope with continuous combat. Common symptoms include flashbacks, avoidant behaviors, and depression.

A method is provided for treatment of a depressive disorder, depression, or depressive illness, comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject. Examples of such disorders include major depression, MDD, drug-resistant depression, dysthymia and bipolar disorder.

In an embodiment, a method is provided for treatment of a mood disorder, or an affective disorder comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject.

Examples of a mood disorder or a affective disorder include major depressive disorder (MDD); bipolar disorder; anhedonia; dysthymia; major depression, Psychotic major depression (PMD), or psychotic depression; postpartum depression; seasonal affective disorder (SAD); and catatonic depression is a rare and severe form of major depression involving disturbances of motor behavior and other symptoms.

The terms "anhedonia" and "anhedonic symptom" are used interchangeably and is defined as the inability to experience pleasure from activities usually found enjoyable, e.g. exercise, hobbies, music, sexual activities or social interactions. The terms"anhedonia" and"anhedonic symptom" are closely related to criterion of"depressive disorder with melancholic features" which is defined in DSM-5 as melancholic depression characterized by a loss of pleasure in most or all activities, a failure of reactivity to pleasurable stimuli, a quality of depressed mood more pronounced than that of grief or loss, a worsening of symptoms in the morning hours, early morning waking, psychomotor retardation, excessive weight loss, or excessive guilt. The term"treatment of depressive disorder with melancholic features" comprises treatment of both the depressive disorder and melancholic features associated herewith. In one embodiment, the mood disorder is anhedonia. In one embodiment, the mood disorder is major depression. In one embodiment, the mood disorder is seasonal affective disorder (SAD).

A method is provided for treatment of an affective disorder, comprising administering to a subject in need thereof an effective amount of a compound of formula I as described herein, or a pharmaceutically acceptable salt thereof, or a pharmaceutical composition comprising the same, at a frequency and for a duration sufficient to provide a beneficial effect to the subject. Affective disorders such as disorders of stress, mood, and behavioral disorders, including stress-related affective disorders, obsessive compulsive disorder, autistic spectrum disorders, Personality disorders, ADHD, panic attacks and the like. As used herein,"autistic spectrum disorders" and "Autism spectrum disorders" are used interchangeably and refer to autism, monogenetic causes of autism such as synaptophathies, e.g., Rett syndrome, Fragile X syndrome, Angelman syndrome and the like.

A method is provided for treatment of Anger, Aggression or Aggressive Disorder, or Impulse Control Disorders comprising administering to a subject in need thereof an effective amount of a compound of formula I as described herein, or a pharmaceutically acceptable salt thereof, or a pharmaceutical composition comprising the same, at a frequency and for a duration sufficient to provide a beneficial effect to the subject. Examples of Anger, Aggression or Aggressive Disorder, or Impulse Control Disorders include, but are not limited to, inappropriate aggression, aggressive behavior, aggression related to social isolation, for treatment of interpersonal violence co-occurring with such illness as ADHD, autism, bipolar disorder, emotional disorders, disorders of memory and/or cognition and cognitive disorders (such as Alzheimer's disease, Parkinson's disease, Huntington's disease and the like), and addictive disorder/substance abuse.

A method is provided for treatment of Intermittent Explosive Disorder (sometimes abbreviated as IED) comprising administering to a subject in need thereof an effective amount of a compound of formula I as described herein, salt thereof, or a pharmaceutical composition comprising the same, at a frequency and for a duration sufficient to provide a beneficial effect to the subject. Intermittent explosive disorder is a behavioral disorder characterized by explosive outbursts of anger and violence, often to the point of rage, that are disproportionate to the situation at hand (e.g., impulsive screaming triggered by relatively inconsequential events). Impulsive aggression is unpremeditated, and is defined by a disproportionate reaction to any provocation, real or perceived. Some individuals have reported affective changes prior to an outburst (e.g., tension, mood changes, energy changes, etc.). The disorder is currently categorized in the Diagnostic and Statistical Manual of Mental Disorders (DSM-5) under the "Disruptive, Impulse-Control, and Conduct Disorders" category. The disorder itself is not easily characterized and often exhibits comorbidity with other mood disorders, particularly bipolar disorder. Individuals diagnosed with LED report their outbursts as being brief (lasting less than an hour), with a variety of bodily symptoms (sweating, stuttering, chest tightness, twitching, palpitations) reported by a third of one sample. Aggressive acts are frequently reported accompanied by a sensation of relief and in some cases pleasure, but often followed by later remorse.

A method is provided for treatment of a Schizophrenia spectrum disorders, comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject. Examples of Schizophrenia spectrum disorders include schizophrenia, schizoaffective disorder, psychotic states and memory disorders.

A method is provided for treatment of a circadian rhythm related disorders, comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject. Circadian rhythm sleep disorders are caused by desynchronization or misalignment between internal sleep-wake rhythms (body clock) and the external light-darkness cycle. Circadian rhythm disorders (sometimes also referred to as phase shift disorders) include sleep disorders associated with jet lag, shift work, or altered sleep phase types, resulting in sleep drifting later each day, abnormal nigh sleep patterns, and/or difficulty staying awake during the day. The cause may be internal (e.g., delayed or advanced sleep phase syndrome, or Non-24-h sleep-wake syndrome) or external (e.g., jet lag, shift work). If the cause is external, other circadian body rhythms, including temperature and hormone secretion, can become out of sync with the light-darkness cycle (external desynchronization) and with one another (internal desynchronization); in addition to insomnia and excessive sleepiness, these alterations may cause nausea, malaise, irritability, and depression. Risk of cardiovascular and metabolic disorders may also be increased. Compounds of the invention are useful for treating circadian rhythm-related disorders, such as depression, jet-lag, work-shift syndrome, sleep disorders, glaucoma, reproduction, cancer, premenstrual syndrome, immune disorders, inflammatory articular diseases and neuroendocrine disorders, Non-24 Hour Disorder.

The compounds according to the invention may also be used in the treatment or prevention of Neuropsychiatric Disorders such as anorexia nervosa, bulimia, mood disorders, depression, anxiety, sleeping disorders, addictive disorders, panic attacks, phobias, obsession, pain-perception disorders (fibromyalgia), dependency on a substance, hemorrhagic stress, muscular spasms and hypoglycemia. Addictive disorder, including disorders related to substance abuse or addiction, and compulsive behavior.

The compounds according to the invention may also be used in the treatment or prevention of chronic stress states such as immunodepression, fertility disorders and dysfunctions of the hypothalamopituitaryadrenal axis.

The compounds according to the invention can also be used in the treatment of disorders such as primary or secondary dysmenorrhea, premature labor or endometriosis, male or female sexual dysfunction, hypertension, chronic heart failure, inappropriate secretion of vasopressin, liver cirrhosis and nephrotic syndrome.

The compounds according to the invention can also be used in the treatment or prevention of any pathology resulting from stress, such as fatigue and its syndromes, ACTH-dependent disorders, cardiac disorders, pain, modifications in gastric emptying, in fecal excretion (colitis, irritable bowel syndrome or Crohn's disease) or in acid secretion, hyperglycemia, immunosuppression, inflammatory processes (rheumatoid arthritis and osteoarthritis), multiple infections, septic shock, cancers, asthma, psoriasis and allergies.

The compounds according to the invention may also be used as psychostimulants, bringing about an increase in consciousness/alertness and/or in emotional reactivity towards the environment and making adaptation easier.

The compounds according to the present invention can be used in healing, in analgesia, in anxiolysis, in the prevention of pain, in the prevention of anxiety, depression, schizophrenia, autism or obsessive-compulsive syndrome, in maternal behavior (facilitation of recognition and acceptance of the mother by the child) and social behavior, memory; regulation of food and drink intake, dependence on drugs, withdrawal and sexual motivation; hypertension, hyponatremia, cardiac insufficiency, atherosclerosis, angiogenesis, the proliferation of tumors, Kaposi's sarcoma, to regulate the storage of fat by the adipocyte, to control hyperlipidemia, triglyceridemia and metabolic syndrome.

The compounds according to the invention can also be used in the treatment of cancers, such as small cell lung cancers or breast cancers; hyponatremic encephalopathy; pulmonary syndrome; Meniere's disease; ocular hypertension; glaucoma; cataracts; obesity; type-I and type-II diabetes; atherosclerosis; metabolic syndrome; hyperlipidemia; insulin resistance; or hypertriglyceridemia; in post-operative treatments, in particular after abdominal surgery; autism; hypercortisolemia; hyperaldosteronemia; pheochromocytoma; Cushing's syndrome; preeclampsia; disorders of micturition; or premature ejaculation.

A certain embodiment of the invention refers to the use of a compound of formula I as described herein for the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

A certain embodiment of this invention refers to the use of a compound of formula I as described herein for the manufacture of a medicament for acting peripherally and centrally in the conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

A certain embodiment of this invention refers to a compound of formula I as described herein for the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

A certain embodiment of this invention refers to a compound of formula I as described herein for the use as therapeutically active substance acting peripherally and centrally in the conditions of inappropriate secretion of vasopressin anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

Also disclosed is a method for the use of a compound as described herein, which is acting peripherally and centrally in the conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag, which method comprises administering said compound of formula I to a human being or animal.

Also disclosed is a method for the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag, which method comprises administering a therapeutically effective amount of a compound of formula I.

The synthesis of the compound of formula I can, for example, be accomplished according to the following scheme. 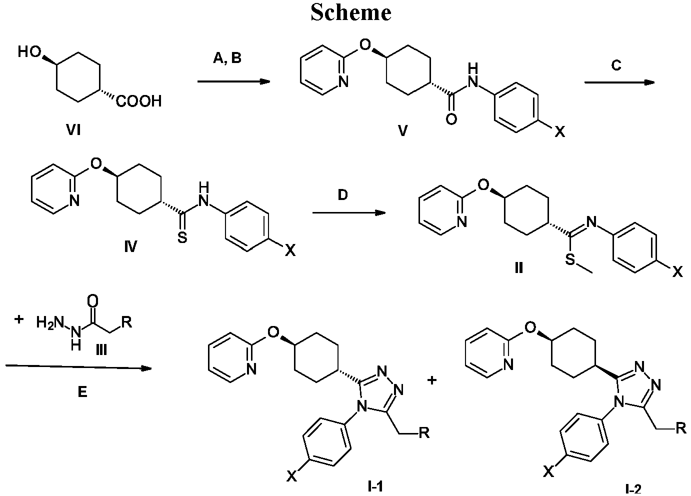
**Step A:** Reaction of trans-4-hydroxycyclohexanecarboxylic acid **IV** with 2-chloropyridine or 2-bromopyridine can be accomplished with an additional base such as sodium tert-pentoxide, sodium tert-butoxide, potassium tert-butoxide, sodium hydride or the like in a suitable solvent such as N-methyl-2-pyrrolidinone (NMP), dimethylacetamide (DMA), dimethylformamide (DMF), tetrahydrofuran (THF) or dimethoxyethane at 20°C to 140°C for 1 h to 120 h. Convenient conditions are heating of trans-4-hydroxycyclohexanecarboxylic acid with 2-chloropyridine and sodium tert-pentoxide in N-methyl-2-pyrrolidinone at 90°C for 96 h.
**Step B:** The amide coupling with the aniline can be accomplished by transforming the acid into an acid chloride by using oxalyl chloride, thionyl chloride or phosphorus oxychloride followed by addition of 4-chloroaniline and a base such as triethylamine, diisopropylethylamine or N-methylmorpholine in various solvents such as dichloromethane, 1,2-dichloroethane, diethyl ether, dioxane, tetrahydrofuran or tert.-butyl methyl ether at -20°C to 70°C for 1 to 24 h. Alternatively, acylation can be accomplished by a coupling reaction between 4-chloroaniline and the carboxylic acid in the presence of a coupling reagent such as DCC, EDC, TBTU or HATU in the presence of an organic base such as triethylamine, diisopropylethylamine or N-methylmorpholine in various solvents such as dichloromethane, 1,2-dichloroethane, diethyl ether, dioxane, tetrahydrofuran or tert.-butyl methyl ether at 0°C to 60°C.
   Convenient conditions are the reaction of the acid with oxalyl chloride in dichloromethane 0-25 °C for 2.5 h followed by reaction of the acid chloride intermediate with the aniline at 0-25°C for 1.5 h.
   The steps A and B can also be reversed.
**Step C:** Formation of the thioamide **IV** can be accomplished by reaction of the amide **V** with a thionation reagent such as Lawesson's reagent or phosphorous pentasulfide in a suitable solvent such as tetrahydrofuran, toluene, xylene, dioxane or 1,2-dimethoxyetane at 20°C to 140°C for 1 h to 24 h.
   Convenient conditions are the use of Lawesson's reagent in tetrahydrofuran at 65°C for 3h.
**Step D:** Formation of the S-methylthioimidate **II** can be accomplished by reaction of the thioamide **IV** with a methylation reagent such as methyl iodide, dimethyl sulfate or methyl 4-methylbenzenesulfonate and an inorganic base such as potassium tert-butoxide, sodium tert-butoxide, sodium hydride or the like in a suitable solvent such as tetrahydrofuran, dioxane, dimethylformamide or dimethylacetamide at 20°C to 70°C for 1 h to 24 h.
   Convenient conditions are the use of methyl 4-methylbenzenesulfonate and potassium tert-butoxide in tetrahydrofuran at room temperature for 1h.
**Step E:** Formation of the triazoles **I-1** and **I-2** can be accomplished by reaction of a hydrazide **III,** the S-methylthioimidate **II** and an acid such as trifluoroacetic acid in a suitable solvent such as n-butanol, n-propanol or 1,4-dioxane at 50°C to 130°C for 1 h to 24 h. As major product of this cyclization or as the only product of the reaction the trans-isomer **I-1** is formed, and in some cases in addition as minor product the cis-isomer **I-2** is formed. If both isomers **I-1** and **I-2** are formed they can be separated by column chromatography or HPLC. Convenient conditions are heating the reactants **II** and **III** with trifluoroacetic acid in n-butanol at 120°C for 2h followed by chromatographic separation.

If a cis-configurated compound I is desired instead of the trans-configurated compound I, then a cis-configurated intermediate, available from cis-4-hydroxycyclohexanecarboxylic acid in a similar way as described can be used as starting material instead of the trans-4-hydroxycyclohexanecarboxylic acid. The minor isomer which may be formed through the synthesis can be removed by standard purification steps as described in the experimental procedures, namely column chromatography or crystallization.

The corresponding pharmaceutically acceptable salts with acids can be obtained by standard methods known to the person skilled in the art, e.g. by dissolving the compound of formula I as described herein in a suitable solvent such as e.g. dioxane or THF and adding an appropriate amount of the corresponding acid. The products can usually be isolated by filtration or by chromatography. The conversion of a compound of formula I into a pharmaceutically acceptable salt with a base can be carried out by treatment of such a compound with such a base. One possible method to form such a salt is e.g. by addition of 1/n equivalents of a basic salt such as e.g. M(OH)ₙ, wherein M = metal or ammonium cation and n = number of hydroxide anions, to a solution of the compound in a suitable solvent (e.g. ethanol, ethanol-water mixture, tetrahydrofuran-water mixture) and to remove the solvent by evaporation or lyophilization.

Insofar as their preparation is not described in the examples, the compounds of formula I as well as all intermediate products can be prepared according to analogous methods or according to the methods set forth herein. Starting materials are commercially available, known in the art or can be prepared by methods known in the art or in analogy thereto.

It will be appreciated that the compounds of general formula I in this invention may be derivatised at functional groups to provide derivatives which are capable of conversion back to the parent compound *in vivo.*

### Pharmacological Tests

The compounds of the present invention exhibit V1a activity. They are selective inhibitors of the V1a receptor and are therefore likely to have a low potential to cause unwanted off-target related side-effects. The V1a activity may be detected as described below.

The human V1a receptor was cloned by RT-PCR from total human liver RNA. The coding sequence was subcloned in an expression vector after sequencing to confirm the identity of the amplified sequence. To demonstrate the affinity of the compounds from the present invention to the human V1a receptor binding studies were performed. Cell membranes were prepared from HEK293 cells transiently transfected with the expression vector and grown in 20 liter fermenters with the following protocol.

50g of cells are re-suspended in 30ml freshly prepared ice cold Lysis buffer (50mM HEPES, 1mM EDTA, 10mM MgCh adjusted to pH= 7.4 + complete cocktail of protease inhibitor (Roche Diagnostics)). Homogenized with Polytron for 1min and sonicated on ice for 2x 2 minutes at 80% intensity (Vibracell sonicator). The preparation is centrifuged 20 min at 500 g at 4°C, the pellet is discarded and the supernatant centrifuged 1hour at 43'000g at 4°C (19'000rpm). The pellet is re-suspended in 12.5 ml Lysis buffer+12.5ml Sucrose 20% and homogenized using a Polytron for 1-2 min. The protein concentration is determined by the Bradford method and aliquots are stored at -80°C until use. For binding studies 60mg Yttrium silicate SPA beads (Amersham) are mixed with an aliquot of membrane in binding buffer (50 mM Tris, 120mM NaCl, 5 mM KCl, 2 mM CaCl₂, 10 mM MgCl₂) for 15 minutes with mixing. 50µl of bead/membrane mixture is then added to each well of a 96 well plate, followed by 50µl of 4 nM 3H-Vasopressin (American Radiolabeled Chemicals). For total binding measurement 100µl of binding buffer are added to the respective wells, for non-specific binding 100µl of 8.4mM cold vasopressin and for compound testing 100µl of a serial dilution of each compound in 2%DMSO. The plate is incubated 1h at room temperature, centrifuged 1 min at 1000g and counted on a Packard Top-Count. Non-specific binding counts are subtracted from each well and data is normalized to the maximum specific binding set at 100%. To calculate an IC 50 the curve is fitted using a non-linear regression model (XLfit) and the Kᵢ is calculated using the Cheng-Prussoff equation.

The following representative data show the antagonistic activity against human V₁ₐ receptor of compounds according to present invention.

**Table 1: pKi values of selected examples**

| **Ex #** | **pKi (hV1a)** | **Ex #** | **pKi (hV1a)** | **Ex #** | **pKi (hV1a)** | **Ex #** | **pKi (hV1a)** | **Ex #** | **pKi (hV1a)** |
|---|---|---|---|---|---|---|---|---|---|
| 1 | 9.77 | 3 | 9.64 | 5 | 9.82 | 7 | 8.44 | 9 | 8.10 |
| 2 | 9.64 | 4 | 9.46 | 6 | 9.06 | 8 | 8.44 | 10 | 8.04 |

### Pharmaceutical Compositions

The compounds of formula I and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compounds of formula I and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also provided by the present invention, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration, the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, in particular 1-100 mg, of a compound of formula I. Examples of compositions according to the invention are:

### Example A

Tablets of the following composition are manufactured in the usual manner:

**Table 2: possible tablet composition**

| ingredient | mg/tablet | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 3: possible capsule ingredient composition**

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I, lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 4: possible soft gelatin capsule ingredient composition**

| ingredient | mg/capsule |
|---|---|
| Compound of formula I | 5 |
| Yellow wax | 8 |
| Hydrogenated Soya bean oil | 8 |
| Partially hydrogenated plant oils | 34 |
| Soya bean oil | 110 |
| Total | 165 |

**Table 5: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 6: possible suppository composition**

| ingredient | mg/supp. |
|---|---|
| Compound of formula I | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula I is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 7: possible injection solution composition**

| ingredient | mg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure

The compound of formula I is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 8: possible sachet composition**

| ingredient | mg/sachet |
|---|---|
| Compound of formula I | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

The following table lists abbreviations used within the present document.

**Table 9: list with abbreviations**

| | |
|---|---|
| brine | saturated sodium chloride solution in water |
| DMAP | 4-(N,N-dimethylamino)-pyridine |
| DMSO | dimethylsulfoxide |
| RT | room temperature |
| THF | tetrahydrofuran |

### Experimental Part

The following examples are provided for illustration of the invention. They should not be considered as limiting the scope of the invention, but merely as being representative thereof.

### Synthesis of the S-Methylthioimidate Intermediate of Formula II

### trans-Methyl N-(4-chlorophenyl)-4-(pyridin-2-yloxy)cyclohexane-1-carbimidothioate

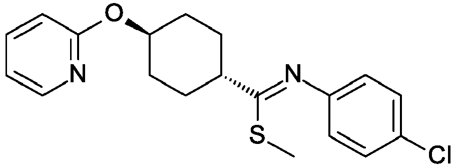

### a) trans-4-(Pyridin-2-yloxy)cyclohexanecarboxylic acid

To a solution of sodium tert-pentoxide (17.3 g, 157 mmol, Eq: 2.44) in N-methyl-2-pyrrolidinone (100 ml) at 90 °C was added trans-4-hydroxycyclohexanecarboxylic acid (CAS 3685-26-5; 9.3 g, 64.5 mmol, Eq: 1.00) dissolved in N-methyl-2-pyrrolidinone (30 ml) in approximately 20 min resulting in a thick suspension. 2-Chloropyridine (8.79 g, 7.26 ml, 77.4 mmol, Eq: 1.2) was added and the reaction mixture was stirred for 96 h at 90 °C. For work-up the reaction mixture was poured into water and set to pH 4-5 by addition of hydrochloric acid (2M, 55.5 ml, 111 mmol, Eq: 1.72). The suspension was stirred for 2 h, then the precipitate was collected by filtration, washed with ice/water (200 ml, in 3 portions) and dried in vacuo to give 9.11 g (64%) of the title compound as off-white solid. MS m/e: 220 (M-H⁺)

### b) trans-N-(4-Chlorophenyl)-4-(pyridin-2-yloxy)cyclohexanecarboxamide

To a solution of trans-4-(pyridin-2-yloxy)cyclohexane-1-carboxylic acid (5 g, 22.6 mmol, Eq: 1) in dichloromethane (200 ml) was added oxalyl chloride (3.44 g, 2.37 ml, 27.1 mmol, Eq: 1.20) at 0-5 °C and subsequently a catalytic amount of DMF. The cooling bath was removed after 30 min and stirring was continued for 2 h. The reaction mixture was concentrated in vacuo and the residue was dissolved in dichloromethane (200 ml). At 0-5°C 4-chloroaniline (2.85 g, 22.4 mmol, Eq: 0.99) was added in one portion and subsequently triethylamine (4.8 g, 6.58 ml, 47.5 mmol, Eq: 2.1). A catalytic amount of DMAP was added and the reaction mixture was stirred for 30 min at 0-5°C. The ice bath was removed and stirring was continued at room temperature for 1 h. For work up the reaction mixture was partitioned between dichloromethane (200 ml) and 0.5 M aqueous sodium hydroxide solution (200 ml and the layers were separated. The aqueous layer was extracted with three 150-ml portions of dichloromethane. The combined organic layers were washed with one 200-ml portion of saturated ammonium chloride solution, two 150-ml portion of water and one 200-ml portion of brine, dried over anhydrous sodium sulfate, filtered and concentrated in vacuo. The residue was triturated in n-heptane ethyl acetate 9:1 (300-ml). The solids was collected by filtration, washed with n-heptane and dried in vacuo to give 6.76 g (86%) of the title compound as off-white solid. MS m/e: 331 (M+ H⁺)

### c) trans-N-(4-Chlorophenyl)-4-(pyridin-2-yloxy)cyclohexanecarbothioamide

A solution of trans-N-(4-chlorophenyl)-4-(pyridin-2-yloxy)cyclohexane-1-carboxamide (6.5 g, 19.6 mmol, Eq: 1) and Lawesson's reagent (4.77 g, 11.8 mmol, Eq: 0.6) in tetrahydrofuran (100 ml) was heated at reflux and stirred for 3 h. For work-up the reaction mixture was concentrated in vacuo and the residue was purified by flash chromatography (silica, 0-100% ethyl acetate in heptane) to yield 4.4g (61%) of the title compound as off-white solid. MS m/e: 347 (M+ H⁺)

### d) trans-Methyl N-(4-chlorophenyl)-4-(pyridin-2-yloxy)cyclohexane-1-carbimidothioate

To a solution of trans-N-(4-chlorophenyl)-4-(pyridin-2-yloxy)cyclohexane-1-carbothioamide (4.4 g, 12.7 mmol, Eq: 1) in tetrahydrofuran (180 ml) was added potassium tert-butoxide (1.45 g, 12.9 mmol, Eq: 1.018) at room temperature. The reaction mixture was stirred for 10 min. Methyl 4-methylbenzenesulfonate (2.42 g, 13 mmol, Eq: 1.023) was added to give a suspension. The stirring was continued at room temperature for 1 h. For work up the reaction mixture was concentrated in vacuo. The residue was partitioned between dichloromethane (200 ml) and water (150 ml) and the layers were separated. The aqueous layer was extracted with one 200-ml portions of dichloromethane. The combined organic layers were washed with one 150-ml portion of water, one 125-ml portion of brine, dried over anhydrous sodium sulfate, filtered and concentrated in vacuo. The residue was purified by flash chromatography (silica, 0-15% ethyl acetate in heptane) to give 4.2 g (87%) of the title compound as white solid. MS m/e: 361 (M+H⁺)

### Examples

### General Procedure: Cyclocondensation of N-(4-chlorophenyl)-4-(pyridin-2-yloxy)cyclohexane-1-carbimidothioate with a hydrazide of formula III to a 1,2,4-triazole of formula I

To a solution of trans-methyl N-(4-chlorophenyl)-4-(pyridin-2-yloxy)cyclohexane-1-carbimidothioate (100 mg, 277 µmol, Eq: 1) and the hydrazide of formula III (333 µmol, Eq: 1.2) in n-butanol (1 ml) was added trifluoroacetic acid (49 mg, 32.9 µl, 429 µmol, Eq: 1.55) at 50 °C. The reaction mixture was heated at 120 °C for 2 h. After cooling to room temperature, the reaction mixture was concentrated in vacuo and purified by column chromatography (silica, 0-20% isopropanol in dichloromethane) or preparative (HPLC column Gemini NX, 12 nm, 5 µm, 100 x 30 mm, water/acetonitrile/formic acid or water/acetonitrile/triethylamine). The major product was always the trans-isomer, however smaller amounts of the cis-isomer could be obtained in some cases by this chromatographic separation as well. The obtained solids were further purified by recrystallisation from ethyl aceate to yield the title compound.

### Example 1

### trans-3-[[4-(4-Chlorophenyl)-5-(4-pyridin-2-yloxycyclohexyl)-1,2,4-triazol-3-yl] methyl]-5-methyl-1,2-oxazole

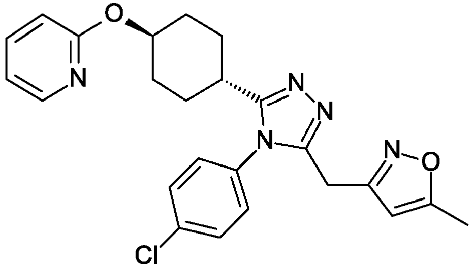

The title compound was obtained as white solid in 23% yield according to the General Procedure.
Hydrazide: 2-(5-methylisoxazol-3-yl)acetohydrazide (CAS 934172-41-5)
MS m/e: 450 (M+H⁺)

### Example 2

### trans-3-[[4-(4-Chlorophenyl)-5-(4-pyridin-2-yloxycyclohexyl)-1,2,4-triazol-3-yl] methyl]-1,2-oxazole

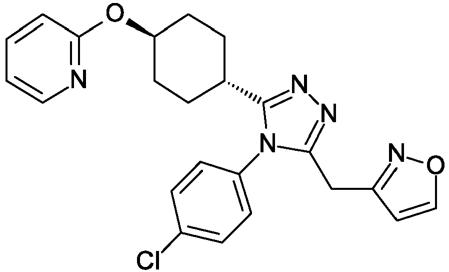

The title compound was obtained as white solid in 29% yield according to the General Procedure.
Hydrazide: 2-(isoxazol-3-yl)acetohydrazide (CAS 934172-86-8)
MS m/e: 436 (M+H⁺)

### Example 3

### trans-5-[[4-(4-Chlorophenyl)-5-(4-pyridin-2-yloxycyclohexyl)-1,2,4-triazol-3-yl]methyl]-1,2-oxazole

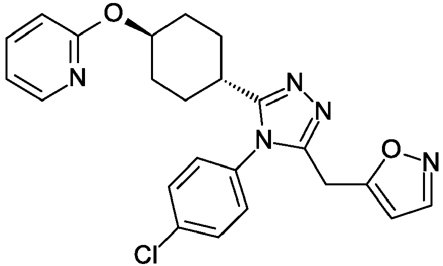

The title compound was obtained as off-white solid in 52% yield according to the General Procedure.
Hydrazide: 2-(isoxazol-5-yl)acetohydrazide (CAS 934172-37-9)
MS m/e: 436 (M+H⁺)

### Example 4

### trans-2-[4-[4-(4-Chlorophenyl)-5-(pyrazol-1-ylmethyl)-1,2,4-triazol-3-yl]cyclohexyl] oxypyridine

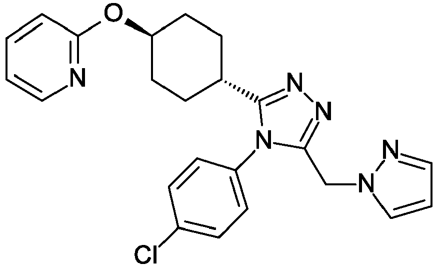

The title compound was obtained as white solid in 23% yield according to the General Procedure.
Hydrazide: 2-(1H-pyrazol-1-yl)acetohydrazide (CAS 934175-49-2)
MS m/e: 435(M+H⁺)

### Example 5

### trans-2-[4-[4-(4-Chlorophenyl)-5-(triazol-2-ylmethyl)-1,2,4-triazol-3-yl]cyclohexyl]oxypyridine

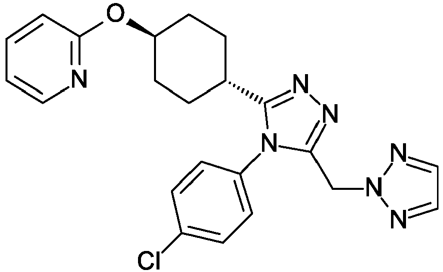

The title compound was obtained as off-white solid in 9% yield (major diastereomer) according to the General Procedure.
Hydrazide: 2-(2H-1,2,3-triazol-2-yl)acetohydrazide (CAS 859154-18-0)
MS m/e: 436 (M+H⁺)

### Example 6

### cis-2-[4-[4-(4-Chlorophenyl)-5-(triazol-2-ylmethyl)-1,2,4-triazol-3-yl]cyclohexyl]oxypyridine

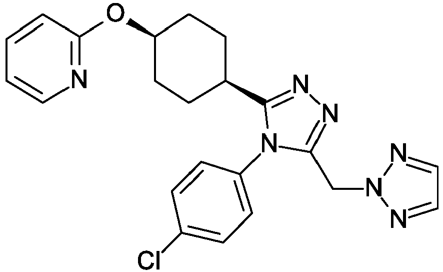

The title compound was obtained as light brown solid in 2% yield (minor diastereomer) according to the General Procedure.
Hydrazide: 2-(2H-1,2,3-triazol-2-yl)acetohydrazide (CAS 859154-18-0)
MS m/e: 436 (M+H⁺)

### Example 7

### trans-2-[4-[4-(4-Chlorophenyl)-5-(triazol-1-ylmethyl)-1,2,4-triazol-3-yl]cyclohexyl]oxypyridine

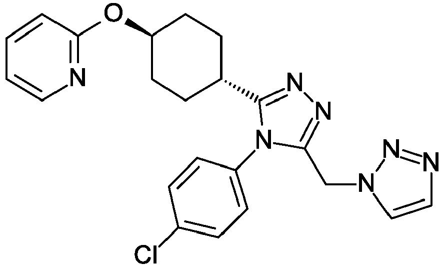

The title compound was obtained as white solid in 30% yield according to General Procedure.
Hydrazide: 2-(1H-1,2,3-triazol-1-yl)acetohydrazide (CAS 4332-47-2)
MS m/e: 436 (M+H⁺)

### Example 8

### trans-2-[4-[4-(4-Chlorophenyl)-5-(imidazol-1-ylmethyl)-1,2,4-triazol-3-yl]cyclohexyl]oxypyridine

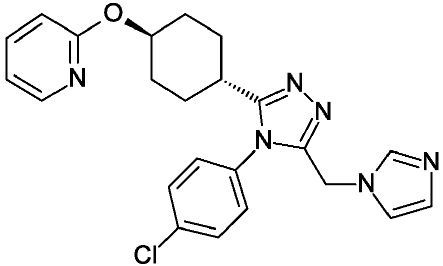

The title compound was obtained as white solid in 19% yield according to General Procedure.
Hydrazide: 2-(1H-imidazol-1-yl)acetohydrazide (CAS 56563-00-9)
MS m/e: 435 (M+H⁺)

### Example 9

### trans-2-[4-[4-(4-Chlorophenyl)-5-(1,2,4-triazol-1-ylmethyl)-1,2,4-triazol-3-yl]cyclohexyl]oxypyridine

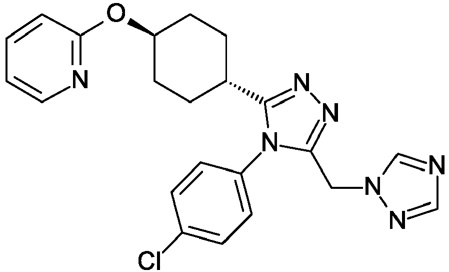

The title compound was obtained as white solid in 18% yield according to General Procedure.
Hydrazide: 2-(1H-1,2,4-triazol-1-yl)acetohydrazide (CAS 56563-02-1)
MS m/e: 436 (M+H⁺)

### Example 10

### trans-2-[4-[4-(4-Chlorophenyl)-5-(tetrazol-1-ylmethyl)-1,2,4-triazol-3-yl]cyclohexyl]oxypyridine

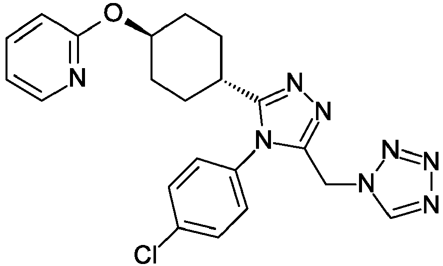

The title compound was obtained as white solid in 9% yield according to General Procedure.
Hydrazide: 2-(1H-tetrazol-1-yl)acetohydrazide (CAS 81548-04-1)
MS m/e: 437 (M+H⁺)

ⁱ Robben, et al. (2006). Am J Physiol Renal Physiol. 291, F257-70, "Cell biological aspects of the vasopressin type-2 receptor and aquaporin 2 water channel in nephrogenic diabetes insipidus"
ⁱⁱ Neumann (2008). J Neuroendocrinol. 20, 858-65, "Brain oxytocin: a key regulator of emotional and social behaviours in both females and males"
ⁱⁱⁱ Ebner, et al. (2002). Eur J Neurosci. 15, 384-8., "Forced swimming triggers vasopressin release within the amygdala to modulate stress-coping strategies in rats"
^{iv} Kendler, et al. (2003). Arch Gen Psychiatry. 60, 789-96, "Life Event Dimensions of Loss, Humiliation, Entrapment, and Danger in the Prediction of Onsets of Major Depression and Generalized Anxiety"
^{v} Regier, et al. (1998). Br J Psychiatry Suppl. 24-8, "Prevalence of anxiety disorders and their comorbidity with mood and addictive disorders"
^{vi} Bielsky, et al. (2004). Neuropsychopharmacology. 29, 483-93, "Profound impairment in social recognition and reduction in anxiety-like behavior in vasopressin V1a receptor knockout mice"
^{vii} Landgraf, et al. (1995). Regul Pept. 59, 229-39., "V1 vasopressin receptor antisense oligodeoxynucleotide into septum reduces vasopressin binding, social discrimination abilities, and anxiety-related behavior in rats"
^{viii} Yirmiya, et al. (2006). 11, 488-94, "Association between the arginine vasopressin 1a receptor (AVPR1a) gene and autism in a family-based study: mediation by socialization skills"
^{ix} Thompson, et al. (2004). Psychoneuroendocrinology. 29, 35-48, "The effects of vasopressin on human facial responses related to social communication"
^{x} Raskind, et al. (1987). Biol Psychiatry. 22, 453-62, "Antipsychotic drugs and plasma vasopressin in normals and acute schizophrenic patients"
^{xi} Altemus, et al. (1992). Arch Gen Psychiatry. 49, 9-20, "Abnormalities in the regulation of vasopressin and corticotropin releasing factor secretion in obsessive-compulsive disorder"
^{xii} Genes, Brain and Behavior (2011) 10: 228-235
^{xiii} Curr. Opin. Neurobiol. 19, 231-234 (2009*)*
^{xiv} Kalsbeek, A., E. Fliers, M. A. Hofman, D. F. Swaab and R. M. Buijs. 2010. Vasopressin and the output of the hypothalamic biological clock.
^{xv} Schwartz, W. J., R. J. Coleman and S. M. Reppert. 1983. A daily vasopressin rhythm in rat cerebrospinal fluid. Brain Res 263: 105-12
^{xvi} Groblewski, T. A., A. A. Nunez and R. M. Gold. 1981. Circadian rhythms in vasopressin deficient rats. Brain Res Bull 6: 125-30
^{xvii} Albers, H. E., C. F. Ferris, S. E. Leeman and B. D. Goldman. 1984. Avian pancreatic polypeptide phase shifts hamster circadian rhythms when microinjected into the suprachiasmatic region. Science 223: 833-5
^{xviii} Yoshiaki Yamaguchi, Toru Suzuki, Yasutaka Mizoro, Hiroshi Kori, Kazuki Okada, Yulin Chen, Jean-Michel Fustin, Fumiyoshi Yamazaki, Naoki Mizuguchi, Jing Zhang, Xin Dong, Gozoh Tsujimoto, Yasushi Okuno, Masao Doi, Hitoshi Okamura. Mice Genetically Deficient in Vasopressin V1a and V1b Receptors Are Resistant to Jet Lag. (2013) Science, 342: 85-90

## Claims

1. A compound of formula I 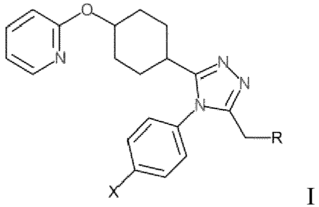 wherein
R is a heteroaryl ring, unsubstituted or substituted with H, halogen, alkyl, haloalkyl, cyano and hydroxyl;
X is H, halogen, alkyl or haloalkyl;
or a pharmaceutically acceptable salt thereof.

2. The compound according to Claim 1, or a pharmaceutically acceptable salt thereof, wherein R is a heteroaryl ring comprising 1-to-4 N and 0-to-1 O, unsubstituted or substituted with H, halogen, alkyl, haloalkyl, cyano and hydroxyl.

3. The compound according to any one of claims 1 to 2, or a pharmaceutically acceptable salt thereof, wherein R is a 5-membered heteroaryl ring comprising 1-to-4 N and 0-to-1 O, unsubstituted or substituted with H, halogen, alkyl, haloalkyl, cyano and hydroxyl.

4. The compound according to any one of claims 1 to 3, or a pharmaceutically acceptable salt thereof, wherein R is a 5-membered heteroaryl ring comprising 1-to-4 N and 0-to-1 O, unsubstituted or substituted with alkyl.

5. The compound according to any one of claims 1 to 4, or a pharmaceutically acceptable salt thereof, wherein R is a 5-membered heteroaryl ring comprising 1 N and 1 O either unsubstituted or substituted with methyl, or unsubstituted heteroaryl ring comprising 1-to-4 N.

6. The compound according to any one of claims 1 to 5, or a pharmaceutically acceptable salt thereof, wherein R is methylisoxazolyl, isoxazolyl, pyrazolyl, triazolyl, imidazolyl, or tetrazolyl.

7. The compound according to any one of claims 1 to 6, or a pharmaceutically acceptable salt thereof, wherein X is halogen.

8. A compound according to claim 1-7, wherein:
R is a 5-membered heteroaryl ring comprising 1 N and 1 O either unsubstituted or substituted with methyl, or unsubstituted heteroaryl ring comprising 1-to-4 N;
X is halogen;
or a pharmaceutically acceptable salt thereof.

9. A compound according to claim 1-8, wherein:
R is methylisoxazolyl, isoxazolyl, pyrazolyl, triazolyl, imidazolyl, or tetrazolyl;
Xis Cl;
or a pharmaceutically acceptable salt thereof.

10. A compound according to claim 1-9, selected from:
trans-3-[[4-(4-chlorophenyl)-5-(4-pyridin-2-yloxycyclohexyl)-1,2,4-triazol-3-yl]methyl]-5-methyl-1,2-oxazole;
trans-3-[[4-(4-chlorophenyl)-5-(4-pyridin-2-yloxycyclohexyl)-1,2,4-triazol-3-yl]methyl]-1,2-oxazole;
trans-5-[[4-(4-chlorophenyl)-5-(4-pyridin-2-yloxycyclohexyl)-1,2,4-triazol-3-yl]methyl]-1,2-oxazole;
trans-2-[4-[4-(4-chlorophenyl)-5-(pyrazol-1-ylmethyl)-1,2,4-triazol-3-yl]cyclohexyl]oxypyridine;
trans-2-[4-[4-(4-chlorophenyl)-5-(triazol-2-ylmethyl)-1,2,4-triazol-3-yl]cyclohexyl]oxypyridine;
cis-2-[4-[4-(4-chlorophenyl)-5-(triazol-2-ylmethyl)-1,2,4-triazol-3-yl]cyclohexyl]oxypyridine;
trans-2-[4-[4-(4-chlorophenyl)-5-(triazol-1-ylmethyl)-1,2,4-triazol-3-yl]cyclohexyl]oxypyridine;
trans-2-[4-[4-(4-chlorophenyl)-5-(imidazol-1-ylmethyl)-1,2,4-triazol-3-yl]cyclohexyl]oxypyridine;
trans-2-[4-[4-(4-chlorophenyl)-5-(1,2,4-triazol-1-ylmethyl)-1,2,4-triazol-3-yl]cyclohexyl]oxypyridine;
trans-2-[4-[4-(4-chlorophenyl)-5-(tetrazol-1-ylmethyl)-1,2,4-triazol-3-yl]cyclohexyl]oxypyridine;
or a pharmaceutically salt thereof.

11. The compound according to any of claims 1 to 10 selected from:
trans-3-[[4-(4-chlorophenyl)-5-(4-pyridin-2-yloxycyclohexyl)-1,2,4-triazol-3-yl]methyl]-5-methyl-1,2-oxazole;
trans-3-[[4-(4-chlorophenyl)-5-(4-pyridin-2-yloxycyclohexyl)-1,2,4-triazol-3-yl]methyl]-1,2-oxazole;
trans-5-[[4-(4-chlorophenyl)-5-(4-pyridin-2-yloxycyclohexyl)-1,2,4-triazol-3-yl]methyl]-1,2-oxazole;
trans-2-[4-[4-(4-chlorophenyl)-5-(pyrazol-1-ylmethyl)-1,2,4-triazol-3-yl]cyclohexyl]oxypyridine;
trans-2-[4-[4-(4-chlorophenyl)-5-(triazol-2-ylmethyl)-1,2,4-triazol-3-yl]cyclohexyl]oxypyridine;
cis-2-[4-[4-(4-chlorophenyl)-5-(triazol-2-ylmethyl)-1,2,4-triazol-3-yl]cyclohexyl]oxypyridine;
or a pharmaceutically acceptable salt thereof.

12. A process for the manufacture of a compound according to any one of claims 1 to 11, or a pharmaceutically acceptable salt thereof, comprising the reaction of a compound of formula II with a compound of formula III, wherein X and R are as defined above.

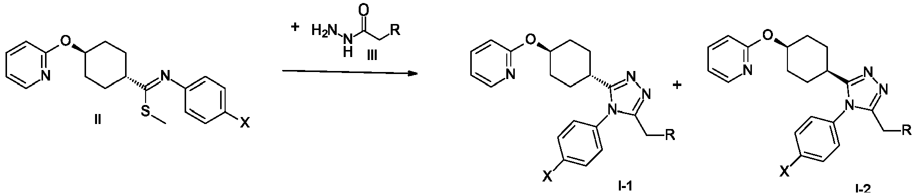

13. A compound according to any of claims 1 to 11, or a pharmaceutically acceptable salt thereof, for use as a therapeutically active substance.

14. A pharmaceutical composition comprising a compound according to any of claims 1 to 11, or a pharmaceutically acceptable salt thereof, and a pharmaceutically acceptable excipient.

15. A compound according to any one of claims 1 to 11, or a pharmaceutically acceptable salt thereof, for use in a therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular j etlag.

## Patentansprüche

1. Verbindung der Formel I 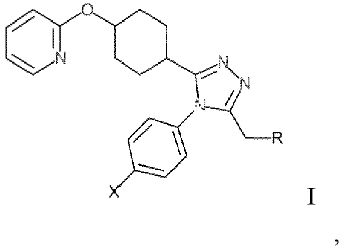 wobei
R ein Heteroarylring ist, der unsubstituiert oder mit H, Halogen, Alkyl, Halogenalkyl, Cyano und Hydroxyl substituiert ist;
X H, Halogen, Alkyl oder Halogenalkyl ist;
oder ein pharmazeutisch unbedenkliches Salz davon.

2. Verbindung nach Anspruch 1 oder ein pharmazeutisch unbedenkliches Salz davon, wobei Rein Heteroarylring, umfassend 1 bis 4 N und 0 bis 1 O, ist, der unsubstituiert oder mit H, Halogen, Alkyl, Halogenalkyl, Cyano und Hydroxyl substituiert ist.

3. Verbindung nach einem der Ansprüche 1 bis 2 oder ein pharmazeutisch unbedenkliches Salz davon, wobei Rein 5-gliedriger Heteroarylring, umfassend 1 bis 4 N und 0 bis 1 O, ist, der unsubstituiert oder mit H, Halogen, Alkyl, Halogenalkyl, Cyano und Hydroxyl substituiert ist.

4. Verbindung nach einem der Ansprüche 1 bis 3 oder ein pharmazeutisch unbedenkliches Salz davon, wobei Rein 5-gliedriger Heteroarylring, umfassend 1 bis 4 N und 0 bis 1 O, ist, der unsubstituiert oder mit Alkyl substituiert ist.

5. Verbindung nach einem der Ansprüche 1 bis 4 oder ein pharmazeutisch unbedenkliches Salz davon, wobei Rein 5-gliedriger Heteroarylring, umfassend 1 N und 1 O, der entweder unsubstituiert oder mit Methyl substituiert ist, oder ein unsubstituierter Heteroarylring, umfassend 1 bis 4 N, ist.

6. Verbindung nach einem der Ansprüche 1 bis 5 oder ein pharmazeutisch unbedenkliches Salz davon, wobei R Methylisoxazolyl, Isoxazolyl, Pyrazolyl, Triazolyl, Imidazolyl oder Tetrazolyl ist.

7. Verbindung nach einem der Ansprüche 1 bis 6 oder ein pharmazeutisch unbedenkliches Salz davon, wobei X Halogen ist.

8. Verbindung nach Anspruch 1-7, wobei:
Rein 5-gliedriger Heteroarylring, umfassend 1 N und 1 O, der entweder unsubstituiert oder mit Methyl substituiert ist, oder ein unsubstituierter Heteroarylring, umfassend 1 bis 4 N, ist;
X Halogen ist;
oder ein pharmazeutisch unbedenkliches Salz davon.

9. Verbindung nach Anspruch 1-8, wobei:
R Methylisoxazolyl, Isoxazolyl, Pyrazolyl, Triazolyl, Imidazolyl oder Tetrazolyl ist;
X Cl ist;
oder ein pharmazeutisch unbedenkliches Salz davon.

10. Verbindung nach Anspruch 1-9, ausgewählt aus:
trans-3-[[4-(4-Chlorphenyl)-5-(4-pyridin-2-yloxycyclohexyl)-1,2,4-triazol-3-yl]methyl]-5-methyl-1,2-oxazol;
trans-3-[[4-(4-Chlorphenyl)-5-(4-pyridin-2-yloxycyclohexyl)-1,2,4-triazol-3-yl]methyl]-1,2-oxazol;
trans-5-[[4-(4-Chlorphenyl)-5-(4-pyridin-2-yloxycyclohexyl)-1,2,4-triazol-3-yl]methyl]-1,2-oxazol;
trans-2-[4-[4-(4-Chlorphenyl)-5-(pyrazol-1-ylmethyl)-1,2,4-triazol-3-yl] cyclohexyl] oxypyridin;
trans-2-[4-[4-(4-Chlorphenyl)-5-(triazol-2-ylmethyl)-1,2,4-triazol-3-yl] cyclohexyl] oxypyridin;
cis-2-[4-[4-(4-Chlorphenyl)-5-(triazol-2-ylmethyl)-1,2,4-triazol-3-yl] cyclohexyl] oxypyridin;
trans-2-[4-[4-(4-Chlorphenyl)-5-(triazol-1-ylmethyl)-1,2,4-triazol-3-yl] cyclohexyl] oxypyridin;
trans-2-[4-[4-(4-Chlorphenyl)-5-(imidazol-1-ylmethyl)-1,2,4-triazol-3-yl] cyclohexyl] oxypyridin;
trans-2-[4-[4-(4-Chlorphenyl)-5-(1,2,4-triazol-1-ylmethyl)-1,2,4-triazol-3-yl] cyclohexyl] oxypyridin;
trans-2-[4-[4-(4-Chlorphenyl)-5-(tetrazol-1-ylmethyl)-1,2,4-triazol-3-yl] cyclohexyl] oxypyridin;
oder ein pharmazeutisch unbedenkliches Salz davon.

11. Verbindung nach einem der Ansprüche 1 bis 10, ausgewählt aus:
trans-3-[[4-(4-Chlorphenyl)-5-(4-pyridin-2-yloxycyclohexyl)-1,2,4-triazol-3-yl]methyl]-5-methyl-1,2-oxazol;
trans-3-[[4-(4-Chlorphenyl)-5-(4-pyridin-2-yloxycyclohexyl)-1,2,4-triazol-3-yl]methyl]-1,2-oxazol;
trans-5-[[4-(4-Chlorphenyl)-5-(4-pyridin-2-yloxycyclohexyl)-1,2,4-triazol-3-yl]methyl]-1,2-oxazol;
trans-2-[4-[4-(4-Chlorphenyl)-5-(pyrazol-1-ylmethyl)-1,2,4-triazol-3-yl] cyclohexyl] oxypyridin;
trans-2-[4-[4-(4-Chlorphenyl)-5-(triazol-2-ylmethyl)-1,2,4-triazol-3-yl] cyclohexyl] oxypyridin;
cis-2-[4-[4-(4-Chlorphenyl)-5-(triazol-2-ylmethyl)-1,2,4-triazol-3-yl] cyclohexyl] oxypyridin;
oder ein pharmazeutisch unbedenkliches Salz davon.

12. Verfahren zur Herstellung einer Verbindung nach einem der Ansprüche 1 bis 11 oder eines pharmazeutisch unbedenklichen Salzes davon, umfassend die Reaktion einer Verbindung der Formel II mit einer Verbindung der Formel III, wobei X und R wie oben definiert sind

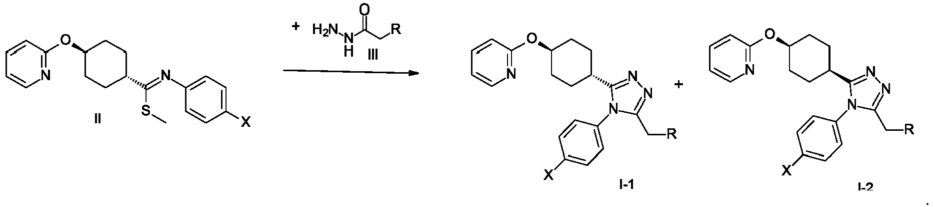

13. Verbindung nach einem der Ansprüche 1 bis 11 oder ein pharmazeutisch unbedenkliches Salz davon zur Verwendung als eine therapeutisch wirksame Substanz.

14. Pharmazeutische Zusammensetzung, umfassend eine Verbindung nach einem der Ansprüche 1 bis 11 oder ein pharmazeutisch unbedenkliches Salz davon und einen pharmazeutisch unbedenklichen Hilfsstoff.

15. Verbindung nach einem der Ansprüche 1 bis 11 oder ein pharmazeutisch unbedenkliches Salz davon zur Verwendung bei einer therapeutischen und/oder prophylaktischen Behandlung von Leiden im Zusammenhang mit unangemessener Sekretion von Vasopressin, Angst, depressiven Störungen, Zwangsstörung, Autismus-Spektrum-Störungen, Schizophrenie, aggressivem Verhalten, sozialer Angststörung, posttraumatischer Belastungsstörung (PTSD), Hirnödem bei Schlaganfall oder traumatischer Hirnverletzung und Schlafstörungen aufgrund von Phasenverschiebung, insbesondere Jetlag.

## Revendications

1. Composé de formule I 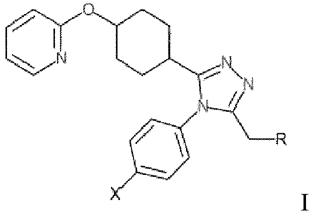 dans lequel
R représente un cycle hétéroaryle, non substitué ou substitué par H, un halogène, un alkyle, un halogénoalkyle, un cyano et un hydroxyle ;
X représente H, un halogène, un alkyle ou un halogénoalkyle ;
ou un sel pharmaceutiquement acceptable de celui-ci.

2. Composé selon la revendication 1, ou un sel pharmaceutiquement acceptable de celui-ci, dans lequel R représente un cycle hétéroaryle comprenant de 1 à 4 N et de 0 à 1 O, non substitué ou substitué par H, un halogène, un alkyle, un halogénoalkyle, un cyano et un hydroxyle.

3. Composé selon l'une quelconque des revendications 1 à 2, ou un sel pharmaceutiquement acceptable de celui-ci, dans lequel R représente un cycle hétéroaryle à 5 chaînons comprenant de 1 à 4 N et de 0 à 1 O, non substitué ou substitué par H, un halogène, un alkyle, un halogénoalkyle, un cyano et un hydroxyle.

4. Composé selon l'une quelconque des revendications 1 à 3, ou un sel pharmaceutiquement acceptable de celui-ci, dans lequel R représente un cycle hétéroaryle à 5 chaînons comprenant de 1 à 4 N et de 0 à 1 O, non substitué ou substitué par un alkyle.

5. Composé selon l'une quelconque des revendications 1 à 4, ou un sel pharmaceutiquement acceptable de celui-ci, dans lequel R représente un cycle hétéroaryle à 5 chaînons comprenant 1 N et 1 O non substitué ou substitué par un méthyle, ou un cycle hétéroaryle non substitué comprenant de 1 à 4 N.

6. Composé selon l'une quelconque des revendications 1 à 5, ou un sel pharmaceutiquement acceptable de celui-ci, dans lequel R représente un méthylisoxazolyle, un isoxazolyle, un pyrazolyle, un triazolyle, un imidazolyle ou un tétrazolyle.

7. Composé selon l'une quelconque des revendications 1 à 6, ou un sel pharmaceutiquement acceptable de celui-ci, dans lequel X représente un halogène.

8. Composé selon la revendication 1 à 7, dans lequel :
R représente un cycle hétéroaryle à 5 chaînons comprenant 1 N et 1 O non substitué ou substitué par un méthyle, ou un cycle hétéroaryle non substitué comprenant de 1 à 4 N ;
X représente un halogène ;
ou un sel pharmaceutiquement acceptable de celui-ci.

9. Composé selon la revendication 1 à 8, dans lequel :
R représente un méthylisoxazolyle, un isoxazolyle, un pyrazolyle, un triazolyle, un imidazolyle ou un tétrazolyle ;
X représente Cl ;
ou un sel pharmaceutiquement acceptable de celui-ci.

10. Composé selon la revendication 1 à 9, choisi parmi :
le trans-3-[[4-(4-chlorophényl)-5-(4-pyridin-2-yloxycyclohexyl)-1,2,4-triazol-3-yl]méthyl]-5-méthyl-1,2-oxazole ;
le trans-3-[[4-(4-chlorophényl)-5-(4-pyridin-2-yloxycyclohexyl)-1,2,4-triazol-3-yl]méthyl]-1,2-oxazole ;
le trans-5-[[4-(4-chlorophényl)-5-(4-pyridin-2-yloxycyclohexyl)-1,2,4-triazol-3-yl]méthyl]-1,2-oxazole ;
la trans-2-[4-[4-(4-chlorophényl)-5-(pyrazol-1-ylméthyl)-1,2,4-triazol-3-yl]cyclohexyl]oxypyridine ;
la trans-2-[4-[4-(4-chlorophényl)-5-(triazol-2-ylméthyl)-1,2,4-triazol-3-yl]cyclohexyl]oxypyridine ;
la cis-2-[4-[4-(4-chlorophényl)-5-(triazol-2-ylméthyl)-1,2,4-triazol-3-yl]cyclohexyl]oxypyridine ;
la trans-2-[4-[4-(4-chlorophényl)-5-(triazol-1-ylméthyl)-1,2,4-triazol-3-yl]cyclohexyl]oxypyridine ;
la trans-2-[4-[4-(4-chlorophényl)-5-(imidazol-1-ylméthyl)-1,2,4-triazol-3-yl]cyclohexyl]oxypyridine ;
la trans-2-[4-[4-(4-chlorophényl)-5-(1,2,4-triazol-1-ylméthyl)-1,2,4-triazol-3-yl]cyclohexyl]oxypyridine ;
la trans-2-[4-[4-(4-chlorophényl)-5-(tétrazol-1-ylméthyl)-1,2,4-triazol-3-yl]cyclohexyl]oxypyridine ;
ou un sel pharmaceutiquement acceptable de ceux-ci.

11. Composé selon l'une quelconque des revendications 1 à 10 choisi parmi :
le trans-3-[[4-(4-chlorophényl)-5-(4-pyridin-2-yloxycyclohexyl)-1,2,4-triazol-3-yl]méthyl]-5-méthyl-1,2-oxazole ;
le trans-3-[[4-(4-chlorophényl)-5-(4-pyridin-2-yloxycyclohexyl)-1,2,4-triazol-3-yl]méthyl]-1,2-oxazole ;
le trans-5-[[4-(4-chlorophényl)-5-(4-pyridin-2-yloxycyclohexyl)-1,2,4-triazol-3-yl]méthyl]-1,2-oxazole ;
la trans-2-[4-[4-(4-chlorophényl)-5-(pyrazol-1-ylméthyl)-1,2,4-triazol-3-yl]cyclohexyl]oxypyridine ;
la trans-2-[4-[4-(4-chlorophényl)-5-(triazol-2-ylméthyl)-1,2,4-triazol-3-yl]cyclohexyl]oxypyridine ;
la cis-2-[4-[4-(4-chlorophényl)-5-(triazol-2-ylméthyl)-1,2,4-triazol-3-yl]cyclohexyl]oxypyridine ;
ou un sel pharmaceutiquement acceptable de ceux-ci.

12. Procédé de fabrication d'un composé selon l'une quelconque des revendications 1 à 11, ou d'un sel pharmaceutiquement acceptable de celui-ci, comprenant la réaction d'un composé de formule II avec un composé de formule III, dans lequel X et R sont tels que définis ci-dessus.

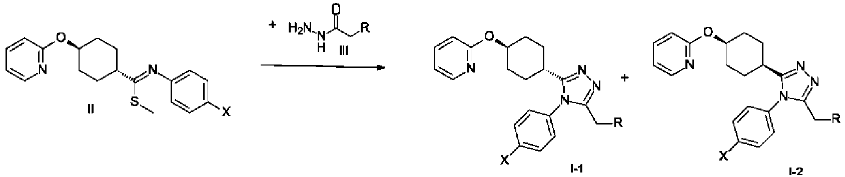

13. Composé selon l'une quelconque des revendications 1 à 11, ou un sel pharmaceutiquement acceptable de celui-ci, pour une utilisation comme substance thérapeutiquement active.

14. Composition pharmaceutique comprenant un composé selon l'une quelconque des revendications 1 à 11, ou un sel pharmaceutiquement acceptable de celui-ci, et un excipient pharmaceutiquement acceptable.

15. Composé selon l'une quelconque des revendications 1 à 11, ou un sel pharmaceutiquement acceptable de celui-ci, pour une utilisation dans un traitement thérapeutique et/ou prophylactique d'affections de la sécrétion inappropriée de la vasopressine, d'une anxiété, de troubles dépressifs, d'un trouble obsessionnel compulsif, de troubles du spectre autistique, d'une schizophrénie, d'un comportement agressif, d'un trouble de l'anxiété sociale, d'un trouble de stress post-traumatique (TSPT), d'oedèmes cérébraux dans un accident vasculaire cérébral ou une lésion cérébrale traumatique et de troubles du sommeil associés à un déphasage, en particulier un décalage horaire.
